(19) Europäisches Patentamt
European Patent Office
Office européen des brevets

(11) Numéro de publication : **0 433 191 B1**

(12) # FASCICULE DE BREVET EUROPEEN

(45) Date de publication du fascicule du brevet : **21.04.93 Bulletin 93/16**

(51) Int. Cl.[5] : **C07C 57/13,** C07C 51/12

(21) Numéro de dépôt : **90420538.2**

(22) Date de dépôt : **11.12.90**

(54) **Procédé de préparation d'acides hexènedioiques-1,6 à partir de butènediol.**

(30) Priorité : **13.12.89 FR 8916755**

(43) Date de publication de la demande : **19.06.91 Bulletin 91/25**

(45) Mention de la délivrance du brevet : **21.04.93 Bulletin 93/16**

(84) Etats contractants désignés : **BE DE ES FR GB IT NL**

(56) Documents cités : **EP-A- 0 124 160**

(73) Titulaire : **RHONE-POULENC CHIMIE**
**25, quai Paul Doumer**
**F-92408 Courbevoie Cédex (FR)**

(72) Inventeur : **Denis, Philippe**
**16, Allée des Troenes**
**F-69150 Decines (FR)**

(74) Mandataire : **Vignally, Noel et al**
**Rhône-Poulenc Chimie Direction de la Propriété Industrielle Centre de Recherches des Carrières B.P. 62**
**F-69192 Saint-Fons Cédex (FR)**

EP 0 433 191 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation d'acides hexènedioïques-1,6. Par acide hexènedioïque-1,6 on entend plus particulièrement l'acide hexène-3 dioïque-1,6. L'acide hexène-3 dioïque-1,6 peut être hydrogéné en acide adipique.

L'acide adipique, l'une des matières premières du nylon 66 est produit avec de forts tonnages et de ce seul fait, toute nouvelle voie d'accès à ce diacide et/ou à ses dérivés présente un intérêt de principe immédiatement perceptible.

La présente invention a plus spécifiquement pour objet un procédé de préparation de l'acide hexène-3 dioïque-1,6 par réaction du monoxyde de carbone et d'au moins un butènediol. Par butènediol on entend le butène-2 diol-1,4, le butène-1 diol-3,4 et leurs mélanges.

Dans la demande de brevet français n° 89/06019 il a été notamment proposé un procédé de préparation de tels diacides par mise en contact du butène-2 diol-1,4, de l'oxyde de carbone et d'un catalyseur à base de palladium, à température élevée et sous une pression supérieure à la pression atmosphérique caractérisé en ce qu'on opère également en présence d'au moins un chlorure d'onium quaternaire d'un élément du groupe VB choisi parmi l'azote et le phosphore, ledit élément étant tétracoordonné à des atomes de carbone, l'azote pouvant être coordonné à deux atomes de phosphore pentavalents.

Il a également été proposé dans cette demande de conduire la réaction dans la N-méthylpyrrolidone-2.

Le procédé en cause qui conduit à des résultats appréciables tant sur le plan de l'activité que sur celui de la sélectivité en produit dicarbonylé linéaire présente cependant l'inconvénient de requérir la présence d'au moins un halogénure d'onium quaternaire au sens indiqué ci-avant, promoteurs relativement onéreux ou peu disponibles et susceptibles de se dégrader lors d'un usage prolongé.

C'est pourquoi il peut s'avérer utile de proposer une alternative audit procédé par laquelle le promoteur organique halogéné n'est plus indispensable et tout ou partie dudit promoteur peut être remplacée par un promoteur halogéné minéral, plus disponible et relativement plus stable lors d'un usage prolongé.

La présente invention a donc pour objet un procédé de préparation d'acides héxènedioïques-1,6 par réaction du monoxyde de carbone et d'au moins un butènediol en présence d'un catalyseur à base de palladium et d'un halogénure caractérisé en ce que la réaction est conduite en présence d'un solvant polaire aprotique basique et en présence d'au moins un halogénure minéral dont le cation est choisi parmi les cations des métaux alcalins et les cations des métaux alcalino-terreux, l'anion halogènure étant choisi parmi le chlorure et le bromure.

Il a en effet été trouvé de manière tout à fait surprenante qu'un tel procédé permet de réaliser la dicarbonylation dans des conditions de pression et de température acceptables à l'échelle industrielle, avec une sélectivité appréciable en produit dicarbonylé linéaire, les proportions de produit monocarbonylé et de produits dicarbonylés branchés étant minimes.

Le procédé selon la présente invention est conduit en présence d'un catalyseur à base de palladium.

Bien que la nature précise de l'espèce (ou des espèces) catalytiquement active(s) dans la réaction en cause ne soit pas totalement élucidée, la Demanderesse a constaté que divers composés du palladium et le palladium métallique sont susceptibles d'être des précurseurs utiles dans la mise en oeuvre du présent procédé.

Parmi les sources de palladium pouvant être mises en oeuvre pour réaliser le procédé faisant l'objet de l'invention, on peut citer :

- . le palladium métallique déposé le cas échéant sur un support, tel que le charbon, l'alumine, ou la silice,
- . $PdCl_2$, $Pd(OAc)_2$
- . les sels ou les complexes $\pi$-allyliques de palladium, dont l'anion coordonné au cation Pd est choisi parmi les anions suivants : carboxylates tels que formiate, acétate, propionate, benzoate ; acétylacétonate, halogénures tels que $Cl^-$ et $Br^-$ et de préférence $Cl^-$;

On recourt avantageusement au chlorure de palladium.

La quantité précise de catalyseur à mettre en oeuvre, qui peut varier dans de larges limites, dépendra avant tout d'un compromis entre l'efficacité souhaitée et la dépense en catalyseur et des autres conditions choisies pour la réaction. En général de bons résultats peuvent être obtenus avec une concentration de palladium dans le milieu réactionnel, comprise entre $10^{-3}$ et 1 mol/l. De préférence, cette concentration est comprise entre $2.10^{-3}$ et $5.10^{-2}$ mol/l.

L'une des caractéristiques essentielles du présent procédé réside dans le fait que la réaction est conduite en présence d'un solvant polaire, aprotique et basique.

Par solvant polaire, aprotique et basique la Demanderesse entend les composés de formule (I) :

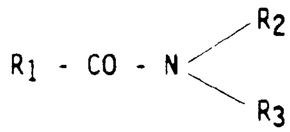 (I)

dans laquelle $R_1$, $R_2$ et $R_3$, identiques ou différents représentent un radical alkyle, un radical cycloalkyle, un radical aralkyle ou un radical aryle monocyclique ayant au plus 10 atomes de carbone, deux radicaux $R_1$, $R_2$ ou $R_3$, pouvant constituer ensemble un radical unique divalent - $(CH_2)_y$ -, y étant un entier compris entre 3 et 12, $R_1$ pouvant en outre représenter un radical

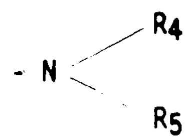 (II)

dans lequel $R_4$ et $R_5$, identiques ou différents représentent un radical alkyle ayant au maximum 4 atomes de carbone.

A titre d'exemple de tels solvants, on peut citer :
la tétraméthylurée, le N,N-diméthylacétamide, le N,N-diéthylacétamide, le N,N-dicyclohexylacétamide, le N,N-diméthylpropionamide, le N-N-diéthylpropionamide, le N,N-diéthyl-n-butyramide, le N,N-diméthylbenzamide, le N,N-dicyclohexylbenzamide, le N,N-diéthyl-m-toluamide, la N-acétylpyrrolidine, la n-acétylpipéridine, la N-(n-butyryl)pipéridine, la N-méthylpyrrolidone-2, la N-éthylpyrrolidone-2, la N-méthylpipéridone-2, le N-méthyl-epsiloncaprolactame.

La N-méthylpyrrolidone-2 convient particulièrement bien à la mise en oeuvre du présent procédé.

En général la quantité d'un tel type de solvants représente au moins 10% en volume du milieu réactionnel ; de bons résultats sont obtenus lorsqu'on en utilise de l'ordre de 20% à 85% en volume.

Bien entendu il est tout à fait possible d'utiliser un mélange de solvants du type précité ou un mélange d'au moins un tel solvant et d'un solvant inerte dans les conditions réactionnelles et n'entrant pas dans la catégorie précitée, tels une cétone, un hydrocarbure aliphatique saturé ou un hydrocarbure aromatique.

De même il est possible d'ajouter au milieu réactionnel un alcanol et d'obtenir alors, tout ou partie, des acides en cause sous forme de leurs diesters correspondants.

Une autre caratéristique essentielle du procédé selon l'invention réside dans le fait que la réaction est conduite également en présence d'un halogénure dont le cation est choisi parmi les cations des métaux alcalins et les cations des métaux alcalino-terreux, l'anion halogénure étant choisi parmi le chlorure et le bromure.

Selon une variante avantageuse du présent procédé on recourt à un chlorure alcalin ou alcalino-terreux.

Il a été constaté que l'effet bénéfique apporté par la présence dans le milieu de carbonylation d'un chlorure ou bromure alcalin ou alcalino-terreux est sensible à partir d'un rapport molaire $Cl^-$(ou $Br^-$)/palladium de 0,5 ; notamment un effet particulièrement intéressant a été constaté lorsque ledit rapport est compris entre 1 et 50, un rapport plus élevé, n'étant toutefois pas nocif pour la réaction.

Bien entendu, on peut mettre en oeuvre dans le cadre de la présente invention un mélange de tels halogénures minéraux ou un mélange d'au moins un halogénure d'onium quaternaire au sens rappelé en tête du présent mémoire.

La réaction pourra être généralement conduite en phase liquide à une température comprise entre 50 et 150°C, de préférence entre 80 et 130°C, sous une pression d'oxyde de carbone comprise entre 20 et 250 bar, de préférence entre 90 et 180 bar.

Des gaz inertes, tels que l'azote, l'argon ou le gaz carbonique, peuvent être présents à côté de l'oxyde de carbone.

En fin de réaction ou du temps imparti à la réaction, on récupère le diacide recherché par tout moyen approprié, par exemple par extraction.

Les exemples ci-après illustrent l'invention; les rendements sont exprimés par rapport au butènediol chargé.

EXEMPLES 1 à 6 ; Essai témoin (a):

Dans un autoclave en acier inoxydable (Hastelloy B2) de 125 cm3, préalablement purgé à l'argon, on introduit :

. 4,4 g (50 mmol) de butène-2 diol-1,4
. 1 mat-g de palladium sous forme de $PdCl_2$
. le cas échéant, 17 mmol de chlorure de calcium (de chlorure de magnésium ou de chlorure alcalin) et
. 25 cm3 de N-méthylpyrrolidone-2 (NMP).

L'autoclave est fermé hermétiquement, placé dans un four agité et raccordé à l'alimentation de gaz sous pression. On purge le réacteur à froid avec de l'oxyde de carbone et on le porte à 100°C. On régule ensuite la pression à 120 bar. Lorsque l'absorption de l'oxyde de carbone est terminée, l'autoclave est refroidi et dégazé.

Le mélange réactionnel est alors estérifié par du méthanol puis analysé par chromatographie en phase gazeuse.

Les conditions particulières ainsi que les résultats obtenus figurent au tableau I ci-après dans lequel t(mn) représente la durée de l'absorption et HD (%) représente le nombre de moles d'acide hexène-3 dioïque formées pour 100 moles de butène-2 diol-1,4 chargées.

Dans l'ensemble des essais le taux de transformation du butène-2 diol-1,4 est de l'ordre de 100%.

TABLEAU I

| Réf. | Nature de l'halogénure | t(mn) | HD(%) |
|---|---|---|---|
| a | néant | 60 | 38 |
| 1 | LiCl | 60 | 71 |
| 2 | KCl | 60 | 45 |
| 3 | NaCl | 60 | 44 |
| 4 | CsCl | 60 | 44 |
| 5 | $MgCl_2,6H_2O$ | 40 | 75 |
| 6 | $CaCl_2$ | 60 | 90 |

Exemple 7 :

On reproduit l'exemple 1 ci-avant en remplaçant la NMP par un volume identique de diméthylacétamide. La durée de l'absorption est de 90 mn ; HD(%) = 78.

Exemple 8 :

On reproduit l'exemple 1 ci-avant en remplaçant LiCl par un mélange de 8,5 mmol de chlorure de tétrabutylphosphonium et de 8,5 mmol de LiCl.
La durée de l'absorption est de 40 mn ; HD(%) = 81.

Exemples 9 à 11 ; essai témoin b :

On réalise une série d'essais selon un mode opératoire analogue à celui décrit précédemment sur une charge renfermant :
- 25 cm3 de solvant ou de mélange de solvants
- 4,4 g (50 mmol) de butène-2 diol-1,4
- 17 mmol de LiCl
- 1 mat-g de palladium sous forme de $PdCl_2$.

La température est de 100°C ; la pression est régulée à 120 bar.
Les conditions particulières et les résultats obtenus figurent dans le tableau II ci-après, dans lequel les conventions utilisées sont celles exposées à propos du Tableau I.

TABLEAU II

| Réf. | NMP (cm3) | co-solvant nature | co-solvant (cm3) | t(mn) | HD (%) |
|---|---|---|---|---|---|
| b | 0 | méthylisobutylcétone | 25 | 75 | 0 |
| 9 | 5 | " | 20 | 70 | 34 |
| 10 | 12,5 | " | 12,5 | 90 | 73 |
| 11 | 12,5 | toluène | 12,5 | 60 | 72 |

**Revendications**

1. Procédé de préparation d'acides hexènedioïques-1,6 par réaction du monoxyde de carbone et d'au moins un butènediol en présence de palladium et d'un halogènure, caractérisé en ce que la réaction est conduite en présence d'un solvant polaire, aprotique, basique et en présence d'au moins un halogénure minéral dont le cation est choisi parmi les cations des métaux alcalins et les cations des métaux alcalino-terreux, l'anion halogénure étant choisi parmi le chlorure et le bromure.

2. Procédé selon la revendication 1, caractérisé en ce que le solvant est choisi parmi les composés de formule (I) :

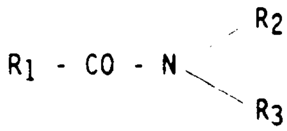

(I)

dans laquelle $R_1$, $R_2$ et $R_3$, identiques ou différents représentent un radical alkyle, un radical cycloalkyle, un radical aralkyle ou un radical aryle monocyclique ayant au plus 10 atomes de carbone, deux radicaux $R_1$, $R_2$ ou $R_3$, pouvant constituer ensemble un radical unique divalent - $(CH_2)_y$ -, y étant un entier compris entre 3 et 12, $R_1$ pouvant en outre représenter un radical

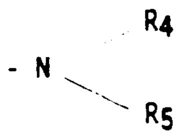

(II)

dans lequel $R_4$ et $R_5$, identiques ou différents représentent un radical alkyle ayant au maximum 4 atomes de carbone.

3. Procédé selon la revendication 1 ou 2 caractérisé en ce que la quantité de solvant représente au moins 10% en volume du milieu réactionnel.

4. Procédé selon l'une quelconque des revendications précédentes caractérisé en ce que le solvant est la N-méthylpyrrolidone-2.

5. Procédé selon l'une quelconque des revendications précédentes caractérisé en ce que l'halogénure minéral est choisi parmi les chlorures.

6. Procédé selon l'une quelconque des revendications précédentes, caractérisé en ce que le rapport molaire de l'anion chlorure (ou bromure) au palladium est compris entre 1 et 50.

7. Procédé selon l'une quelconque des revendications précédentes, caractérisé en ce que la concentration du palladium dans le milieu réactionnel est comprise entre $10^{-3}$ et 1 mol/l.

8. Procédé selon l'une quelconque des revendications précédentes, caractérisé en ce que la température de réaction est comprise entre 50 et 150°C et, de préférence, entre 80 et 130°C.

9. Procédé selon l'une quelconque des revendications précédentes, caractérisé en ce que la pression est comprise entre 20 et 250 bar et, de préférence, entre 90 et 180 bar (9000 et 18000 KPa).

10. Procédé selon l'une quelconque des revendications précédentes, caractérisé en ce que le butènediol est choisi parmi le butène-2 diol-1,4 et le butène-1 diol-3,4 et leurs mélanges.

11. Procédé selon l'une quelconque des revendications précédentes, caractérisé en ce que le palladium est engagé sous forme de chlorure de palladium.

## Patentansprüche

1. Verfahren zur Herstellung von Hexen-1,6-disäuren durch Reaktion von Kohlenmonoxid und mindestens einem Butendiol in Anwesenheit von Palladium und einem Halogenid, dadurch gekennzeichnet, daß die Reaktion in Anwesenheit eines basischen, aprotischen polaren Lösungsmittels und in Anwesenheit von mindestens einem Mineralhalogenid durchgeführt wird, dessen Kation unter den Alkalimetall-Kationen und den Erdalkalimetall-Kationen und dessen Halogenidanion unter Chlorid und Bromid ausgewählt werden.

2. Verfahren nach Anspruch 1, dadurch gekennzeichnet, daß das Lösungsmittel ausgewählt wird unter den Verbindungen der Formel (I)

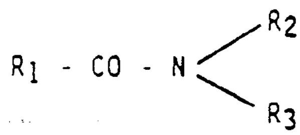

(I)

in der $R_1$, $R_2$ und $R_3$, gleich oder verschieden, einen Alkyl-Rest, einen Cycloalkyl-Rest, einen Aralkyl-Rest oder einen monocyclischen Aryl-Rest mit höchstens 10 Kohlenstoffatomen darstellen, wobei zwei Reste $R_1$, $R_2$ oder $R_3$ zusammen einen einzigen divalenten Rest $-(CH_2)_y-$ bilden können, und y eine ganze Zahl zwischen 3 und 12 ist, wobei $R_1$ außerdem einen Rest (II)

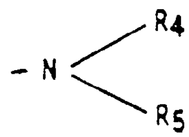

(II)

bedeuten kann, in dem $R_4$ und $R_5$, gleich oder verschieden, einen Alkyl-Rest mit höchstens 4 Kohlenstoffatomen darstellen.

3. Verfahren nach Anspruch 1 oder 2, dadurch gekennzeichnet, daß die Menge des Lösungsmittels mindestens 10 Volumenprozent des Reaktionsmediums beträgt.

4. Verfahren nach irgendeinem der vorstehenden Ansprüche, dadurch gekennzeichnet, daß das Lösungsmittel N-Methyl-2-pyrrolidon ist.

5. Verfahren nach irgendeinem der vorstehenden Ansprüche, dadurch gekennzeichnet, daß das Mineralhalogenid unter den Chloriden ausgewählt wird.

6. Verfahren nach irgendeinem der vorstehenden Ansprüche, dadurch gekennzeichnet, daß das molare Verhältnis von Chlorid-(oder Bromid-)anion zu Palladium zwischen 1 und 50 beträgt.

7. Verfahren nach irgendeinem der vorstehenden Ansprüche, dadurch gekennzeichnet, daß die Konzentration des Palladiums in dem Reaktionsmedium zwischen $10^{-3}$ und 1 Mol/l beträgt.

8. Verfahren nach irgendeinem der vorstehenden Ansprüche, dadurch gekennzeichnet, daß die Temperatur der Reaktion zwischen 50 °C und 150 °C, vorzugsweise zwischen 80 °C und 130 °C, beträgt.

9. Verfahren nach irgendeinem der vorstehenden Ansprüche, dadurch gekennzeichnet, daß der Druck zwischen 20 bar und 250 bar, vorzugsweise zwischen 90 bar und 180 bar (9000 und 18000 kPa), beträgt.

10. Verfahren nach irgendeinem der vorstehenden Ansprüche, dadurch gekennzeichnet, daß das Butendiol ausgewählt wird unter 2-Buten-1,4-diol und 1-Buten-3,4-diol sowie deren Mischungen.

11. Verfahren nach irgendeinem der vorstehenden Ansprüche, dadurch gekennzeichnet, daß das Palladium in Form von Palladiumchlorid eingesetzt wird.

## Claims

1. Process for the preparation of 1,6-hexenedioic acids by reaction of carbon monoxide and of at least one butenediol in the presence of palladium and of a halide, characterised in that the reaction is conducted in the presence of an aprotic, basic polar solvent and in the presence of at least one inorganic halide whose cation is chosen from alkali metal cations and alkaline-earth metal cations, the halide anion being chosen from chloride and bromide.

2. Process according to Claim 1, characterised in that the solvent is chosen from the compounds of formula (I):

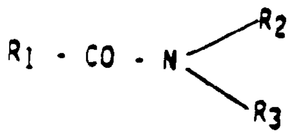

(I)

in which $R_1$, $R_2$ and $R_3$, which are identical or different, denote an alkyl radical, a cycloalkyl radical, an aralkyl radical or a monocyclic aryl radical containing not more than 10 carbon atoms, it being possible for two radicals $R_1$, $R_2$ or $R_3$ to form together a single divalent radical $-(CH_2)_y-$, y being an integer between 3 and 12, it being additionally possible for $R_1$ to denote a radical

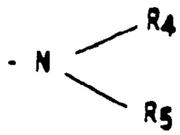

(II)

in which $R_4$ and $R_5$, which are identical or different, denote an alkyl radical containing not more than 4 carbon atoms.

3. Process according to Claim 1 or 2, characterised in that the quantity of solvent represents at least 10 % by volume of the reaction mixture.

4. Process according to any one of the preceding claims, characterised in that the solvent is N-methyl-2-pyrrolidone.

5. Process according to any one of the preceding claims, characterised in that the inorganic halide is chosen from chlorides.

6. Process according to any one of the preceding claims, characterised in that the molar ratio of the chloride (or bromide) anion to palladium is between 1 and 50.

7. Process according to any one of the preceding claims, characterised in that the concentration of palladium in the reaction mixture is between $10^{-3}$ and 1 mol/l.

8. Process according to any one of the preceding claims, characterised in that the reaction temperature is between 50 and 150 °C and, preferably, between 80 and 130°C.

9. Process according to any one of the preceding claims, characterised in that the pressure is between 20 and 250 bar and, preferably, between 90 and 180 bar (9000 and 18,000 kPa).

10. Process according to any one of the preceding claims, characterised in that the butenediol is chosen from 2-butene-1,4-diol and 1-butene-3,4-diol and mixtures thereof.

11. Process according to any one of the preceding claims, characterised in that the palladium is introduced in the form of palladium chloride.